# EUROPEAN PATENT APPLICATION

(11) **EP 2 842 593 A1**
(43) Date of publication of application: **04.03.2015**
(21) Application number: 13290208.1
(22) Date of filing: 03.09.2013
(51) Int. Cl.: A61M 25/02, A61M 25/06

(54) **Access sheath**

(71) Applicant: Coloplast A/S, 3050 Humlebaek (DK)
(72) Inventor: Callede, David, 24200 Sarlat la Caneda (FR); Pascal, Laurent, 24200 Sarlat La Caneda (FR)

(57) **Abstract**

The present invention concerns an (access sheath) access sheath (10) intended for positioning a tool in a working position during an intervention on a male patient, said (access sheath) access sheath (10) comprising a guide tube (11) and a bucket (12). The (access sheath) access sheath of the present invention is characterized in that it comprises a maintaining piece (13) adapted to be fixed to said patient during the use of the (access sheath) access sheath.

## Description

### TECHNICAL FIELD

The present invention concerns an access sheath intended for positioning a tool in a working position during an intervention on a male patient, said access sheath comprising a guide tube and a bucket.

This kind of access sheath is often used together with a tool by surgeons to enable them to make an intervention in areas of the human body which can be difficult to access. These access sheath/tool assemblies are used, among others, in urology. More particularly, the access sheath of the present invention is used for urological interventions on male patients.

Such an access sheath can be used on its own, in particular for inserting tools into an area of the body where an intervention has to take place. The access sheath of the invention can also be part of an assembly further comprising a dilator that may be inserted into the access sheath and a guide for guiding the access sheath during its insertion into the patient's body.

### BACKGROUND ART

When a surgeon wants to gain access to a kidney, if surgical intervention is not an option, he has to introduce an access sheath inside the urethra starting from a natural route of entry, pass the bladder and then go up inside the ureter to reach the kidney.

The positioning of such an access sheath can be performed by several methods. According to a first method, a first radio-opaque guide is driven up to the bladder by the aid of a cystoscope previously introduced inside the bladder. Then, with the aid of the cystoscope, the urethral meatus is targeted in order to insert the guide into the urethra. The guide is a generally sheathed nickel, titanium and/or stainless steel alloy lead.

After the setting up of this first guide, a radio-opaque double channel urethral probe or dilator is engaged by one of its two channels onto the guide and is driven up to the urethra. Through the other channel of the urethral probe, a second guide is inserted until it reaches the urethra. Then the urethral probe is removed, leaving behind only the two guides : a working guide and a security guide, this latter being fixed to the patient.

During these first steps, the radio-opaque components have been visualized to check their positions.

The access sheath being threaded onto a dilator projecting forwards outside the sheath, the working guide is engaged into the access sheath through the dilator channel. The access sheath is driven up to a position between the bladder and the kidney, but nearer the bladder. The dilator and the working guide are removed to leave behind in place only the access sheath, and near it, the security guide that can be used in case of difficulty.

According to a second, more efficient method, the positioning of the access sheath requires the use of only one guide and a dilator comprising at least a hole at its distal end, i.e. the end that is in the area of the intervention when the access sheath is in use. The dilator further comprises an opening connected to the hole by a channel comprising a slit on its periphery. Said opening is positioned outside of the access sheath when the access sheath and the dilator are assembled. Such an access sheath/dilator assembly is described in the patent application WO 2009/127216.

As in the previous embodiment, a guide is driven up to the bladder of the patient. The access sheath/dilator assembly is engaged with the guide, this guide entering the hole at the distal end of the dilator, following the channel and exiting the dilator through the opening.

The access sheath/dilator assembly is positioned by following the guide until it reaches the final position. The dilator is then pulled out of the access sheath. This has the effect of applying strength on the guide which in turn opens the slit of the dilator and releases the guide from the dilator. Thus, the access sheath is ready for use, with the guide following the access sheath on the external side.

Once the access sheath is in the correct position, it is only maintained in this correct position by the resistance from the urethra. The movements made by the surgeon while (s)he is working, for example while removing a kidney stone, as well as the pressure of the urine from the ureter and the bladder will cause the access sheath to move backwards and to leave the correct position.

These movements make the intervention of the surgeon more complex, longer as it may be necessary to reposition the access sheath, and may lead to injuries to the patient, making recovery longer and more painful.

The aim of this invention is to provide a device for ensuring a correct positioning of the access sheath with regard to the patient during an operation. This invention prevents the access sheath from moving with regard to the patent during the operation. This lowers the risk of injuries of the patient and facilitates the task of the surgeon.

According to a particular embodiment of the invention, the access sheath of the invention also facilitates its introduction and positioning.

### DISCLOSURE OF INVENTION

The object of the invention is achieved by a access sheath such as described in the preamble and **characterized in that** said access sheath comprises a maintaining piece adapted to be fixed to said patient during the use of the access sheath.

According to the present invention, the access sheath can be used in cooperation with different kinds of tools such as for example an endoscope, tools for grasping objects, tools for cutting tissues, tools with laser beams... These tools are usually introduced in the access sheath once this access sheath is in place within the body of the patient, an end of said access sheath being placed in the area where the intervention takes place. The access sheath of the invention is fixed to the patient and facilitates the intervention of the surgeon as it prevents the movements of the access sheath during the intervention.

### BRIEF DESCRIPTION OF DRAWING

The present invention and its advantages will be better understood with reference to the enclosed drawing and to the detailed description of specific embodiments, in which :
- Fig. 1 is a partial cross section view of a first embodiment of a access sheath according to the present invention;
- Fig. 2 is a cross section view of a access sheath of the invention according to a variant; and
- Fig. 3 shows another embodiment of the access sheath of the present invention.

### BEST MODES FOR CARRYING OUT THE INVENTION

According to the present invention, the access sheath 10 is intended for receiving a tool that will be used in an area of a body which is difficult to access. This tool can be an endoscope or other devices for taking pictures of the inner part of the patient's body, tools for grasping objects, tools for cutting patient's tissues, tools for delivering drugs, liquids or other products or tools comprising laser beams for example. The access sheath comprises a hollow guide tube 11 for receiving and guiding a tool (not represented). The access sheath further comprises a bucket 12 having a general shape of a funnel with a hole coinciding with the hole of the guide tube. The access sheath further comprises a maintaining piece 13 which has a generally tubular shape.

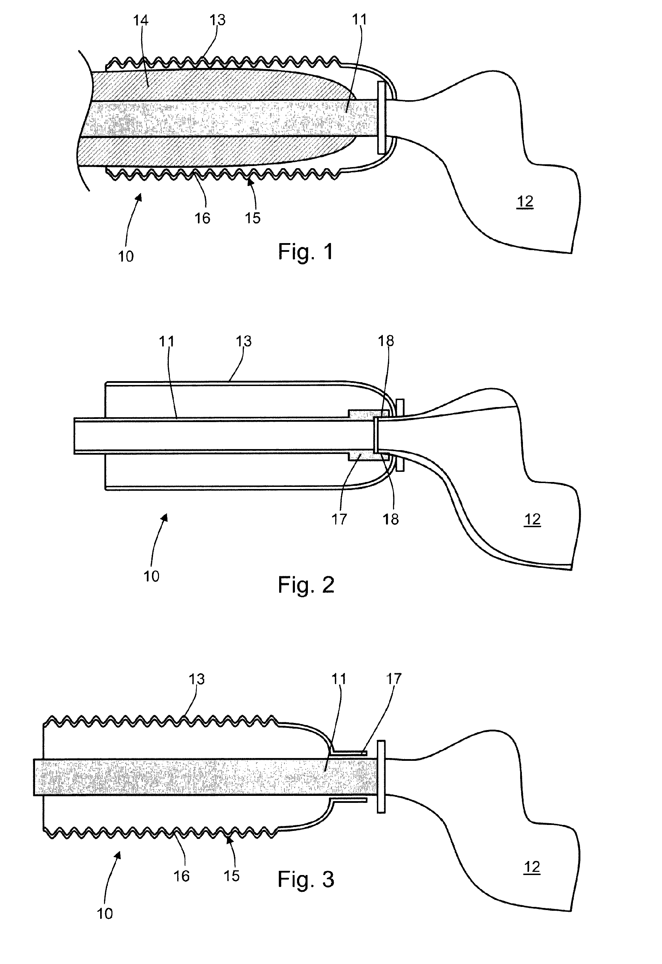

According to the embodiment illustrated by Fig. 1, one end of the maintaining piece 13 is integral with the bucket 12 or with an area of the guide tube 11 close to the bucket. The other end of the maintaining piece 13 is open and is conformed to be able to receive the penis 14 of the patient.

According to a preferred embodiment, the maintaining piece 13 comprises an extensible area 15. This extensible area 15 preferably comprises bellows 16 arranged to enable the modification in length and in diameter of this maintaining piece. This extensible area 15 could also be formed of an elastic material.

For the use of the access sheath illustrated by Fig. 1, when an intervention takes place, the guide tube 11 of the access sheath 10 is introduced in the urethra of the patient. At the end of the introduction of the guide tube 11, the penis 14 of the patient is introduced in the maintaining piece 13 in order to maintain the access sheath 10 in a correct position. The size of the maintaining piece can be adapted by deformation of the bellows 16 so as to ensure an optimal holding. According to a variant, the maintaining piece could be fixed to the penis of the patient by a skin-friendly adhesive. This adhesive could be used in addition to the bellows or instead of the bellows.

According to another embodiment, illustrated in particular by Fig. 2, the maintaining piece 13 can be changed and replaced. In this case, it is possible to choose the size of the maintaining piece 13 to fit the penis of the patient. In this embodiment, the bucket 12 can be reversibly separated from the guide tube.

A proximal end 17 of the guide tube has a recess 18 cooperating with a shoulder 19 formed at a distal end of the bucket. In the embodiment illustrated, the bucket 12 can be separated from the guide tube 11 by pulling this bucket apart from the guide tube.

It is also possible to provide means for assembling the guide tube and the bucket only once, without the possibility of separating these elements once assembled. This can be done for example by deformable hooks. In this case, the access sheath 10 is not assembled before it is used. When a surgeon intends to use the access sheath 10, (s)he chooses the adapted maintaining piece 13 and (s)he assembles the maintaining piece 13, the guide tube 11 and the bucket 12 in such a way that the maintaining piece 13 is held between the guide tube 11 and the bucket 12.

It is also possible to provide a means for assembling and disassembling the access sheath 10 only with a specific tool, by applying a strength over a given threshold or by realising a specific movement (which would be the case for example with a bayonet fitting).

According to the embodiment of Fig. 2, in order to position the maintaining piece 13, the bucket 12 is first separated from the guide tube 11 if these elements are not already separated. The maintaining piece 13 is positioned on the bucket 12 and the maintaining piece 13 plus the bucket 12 are assembled on the guide tube 11. Thus, the maintaining piece is firmly maintained between the guide tube and the bucket. It should be noted that numerous other configurations are possible for maintaining the maintaining piece, the bucket and the guide tube together.

According to another embodiment illustrated by Fig. 3, the maintaining piece 13 can slide along the guide tube 11. In this embodiment, the proximal end 17 of the maintaining piece has a hole whose dimension is slightly greater than the dimensions of the guide tube 11. In a preferred embodiment, the maintaining piece 13 can slide along the guide tube 11, but is not loose.

In this embodiment, the maintaining piece 13 can further be used as a guide during the introduction of the guide tube 11 in the urethra of the patient. The maintaining piece 13 can be slightly rigid in order to maintain the urethra in a rectilinear position. This facilitates the introduction of the guide tube.

Thank to this invention, the access sheath is fixed with regard to the patient. This greatly facilitates the operations made by the surgeon as a substantial proportion of the access sheath does not move. Thus, the intervention can be done more quickly and more accurately, which lowers the risks of injuries and facilitates the recovery of the patient.

## Claims

1. Access sheath (10) intended for positioning a tool in a working position during an intervention on a male patient, said access sheath (10) comprising a guide tube (11) and a bucket (12), **characterized in that** said access sheath (10) comprises a maintaining piece (13) adapted to be fixed to said patient during the use of the access sheath.

2. Access sheath according to claim 1, **characterized in that** the maintaining piece (13) has a tubular shape.

3. Access sheath according to claim 1, **characterized in that** a proximal end of the maintaining piece (13) is integral with at least one of the guide tube (11) or the bucket (12), and a distal end of the maintaining piece (13) is open for receiving a penis (14) of said male patient.

4. Access sheath according to claim 2, **characterized in that** said maintaining piece (13) comprises an extensible area (15).

5. Access sheath according to claim 4, **characterized in that** the extensible area (15) comprises bellows (16).

6. Access sheath according to claim 1, **characterized in that** said guide tube (11) and said bucket (12) comprise means for assembling the guide tube (11) and the bucket (12) together and for blocking the maintaining piece (13) when the guide tube (11) and the bucket (12) are assembled.

7. Access sheath according to claim 6, **characterized in that** said means for assembling the guide tube and the bucket comprises a recess (18) at a proximal end (17) of the guide tube (11).

8. Access sheath according to claim 6, **characterized in that** said means for assembling the guide tube and the bucket comprises a shoulder (19) at a distal end of the bucket (12).

9. Access sheath according to claim 6, **characterized in that** said means for assembling the guide tube and the bucket comprises deformable hooks.

10. Access sheath according to claim 1, **characterized in that** said maintaining piece (13) is adapted to slide on the guide tube (11).

11. Access sheath according to claim 1, **characterized in that** said maintaining piece (13) comprises a skin-friendly adhesive.
